# EUROPEAN PATENT SPECIFICATION

(11) **EP 4 578 398 B1**
(45) Date of publication and mention of the grant of the patent: **14.10.2026**
(21) Application number: 24222830.2
(22) Date of filing: 23.12.2024
(51) Int. Cl.: A61B 6/03, A61B 6/04, A61B 6/00

(54) **INFORMATION PROCESSING APPARATUS, INFORMATION PROCESSING METHOD, AND INFORMATION PROCESSING PROGRAM**
INFORMATIONSVERARBEITUNGSVORRICHTUNG, INFORMATIONSVERARBEITUNGSVERFAHREN UND INFORMATIONSVERARBEITUNGSPROGRAMM
APPAREIL, PROCÉDÉ ET PROGRAMME DE TRAITEMENT D'INFORMATIONS

(30) Priority: 26.12.2023 JP 2023219810
(43) Date of publication of application: 02.07.2025
(73) Proprietor: FUJIFILM Corporation, Tokyo 106-8620 (JP)
(72) Inventor: HAYASHI, Tetsuya, Tokyo, 106-8620 (JP)
(74) Representative: Dehns Germany Partnerschaft mbB

(56) References cited:
- JP-A- 2021 079 013
- US-A1- 2020 218 922
- US-A1- 2020 375 561

## Description

### BACKGROUND OF THE INVENTION

### Technical Field

The present invention relates to an information processing apparatus, an information processing method, and an information processing program.

### Related Art

In recent years, with the advancement of medical equipment, such as a computed tomography (CT) apparatus and a magnetic resonance imaging (MRI) apparatus, three-dimensional images having a higher-quality and a higher-resolution have been used for image diagnosis.

In a case in which imaging of a subject is performed with an imaging apparatus, such as the CT apparatus or the MRI apparatus, in order to determine an imaging range, scout imaging is performed before main imaging for acquiring a three-dimensional image to acquire a two-dimensional image for positioning (scout image). An operator (technician or the like) of the imaging apparatus sets an imaging range during the main imaging while viewing the scout image.

Before the scout imaging, the operator sets the imaging range of the scout imaging in the subject on an examination table. For example, the subject is irradiated with a cross-shaped laser, a scan start position of the scout imaging is set, and a scan end position of the scout imaging is set such that the imaging range corresponding to an imaging part is obtained. In a case of the scout imaging, the scan in the scout imaging is started in a case in which the examination table is moved from an initial position of the examination table to the scan start position, and the scan in the scout imaging ends in a case in which the examination table is moved to the scan end position. The operator sets the imaging range of the main imaging by using the scout image acquired by the scout imaging, and then performs the main imaging to acquire the three-dimensional image.

Here, during setting the imaging range during the scout imaging, the subject is imaged by a camera provided above the examination table, feature points such as both ankles, both waists, both elbows, and both shoulders of the subject are detected, and the imaging range is specified based on the detected feature points. In this case, a trained detection model constructed by training a neural network through machine learning is used for the detection of the feature points.

Meanwhile, a method has been proposed in which, in a case in which a trained model is applied to a medical image, a trained model that performs a plurality of different types of processing is prepared, and a trained model to be used is selected depending on a situation, thereby efficiently processing the medical image (see, for example, JP2021-079013A).

As described above, in a case where the subject is imaged by the camera to perform the positioning of the imaging range, the subject may move on the examination table. In particular, the head of the subject is likely to move. In a case where the detection model detects the feature points from the image captured by the camera, there is a possibility that the feature points cannot be detected with high accuracy in a case where the subject moves. In addition, the subject may be covered with a blanket during imaging, but in this case, since a part of the subject is covered, there is a possibility that the feature points cannot be detected.

US2020375561A1 discloses an information processing apparatus comprising: at least one processor, wherein the processor acquires a camera image generated by capturing a moving image of a subject on an examination table via a camera, detects the plurality of feature points on the subject included in the camera image by using the selected detection model, and specifies an imaging range of the subject based on the plurality of feature points.

### SUMMARY OF THE INVENTION

The present invention has been made in view of the above circumstances, and an object of the present invention is to appropriately detect feature points in a case of setting an imaging range.

According to the present invention, there is provided an information processing apparatus comprising: at least one processor, in which the processor acquires a camera image generated by capturing a moving image of a subject on an examination table via a camera, selects one detection model from among a plurality of detection models including a first detection model that places importance on a frame rate in a case of detecting the feature points and a second detection model that places importance on accuracy in a case of detecting the feature points, which are constructed so as to detect a plurality of feature points on the subject included in the camera image, detects the plurality of feature points on the subject included in the camera image by using the selected detection model, and specifies an imaging range of the subject based on the plurality of feature points.

In the information processing apparatus according to the present invention, the processor may select the detection model in accordance with an imaging part of the subject.

In addition, in the information processing apparatus according to the present invention, the processor may determine detection accuracy of the feature points, specify the imaging range based on the feature points in a case in which the detection accuracy is equal to or higher than a reference, and issue an alert in a case in which the detection accuracy is lower than the reference.

In the information processing apparatus according to the present invention, the processor may detect a movement of the subject based on the camera image, specify the imaging range based on the feature points detected by using the first detection model in a case in which the movement of the subject is equal to or larger than a reference, and specify the imaging range based on the feature points detected by using the second detection model in a case in which the movement of the subject is smaller than the reference.

In the information processing apparatus according to the present invention, the processor may detect the feature points from the camera image by selecting the first detection model, and detect a movement of the subject based on the feature points.

In addition, in the information processing apparatus according to the present invention, the processor may determine detection accuracy of the feature points, specify the imaging range based on the feature points in a case in which the detection accuracy is equal to or higher than a reference, and issue an alert in a case in which the detection accuracy is lower than the reference.

In the information processing apparatus according to the present invention, the processor may detect the feature points from the camera image by selecting the first detection model, determine detection accuracy of the feature points, specify the imaging range based on the feature points detected by using the first detection model in a case in which the detection accuracy is equal to or higher than a first reference, select the second detection model in a case in which the detection accuracy is lower than the first reference, and specify the imaging range based on the feature points detected by using the second detection model.

In the information processing apparatus according to the present invention, the processor may determine detection accuracy of the feature points detected by using the second detection model, specify the imaging range based on the feature points in a case in which the detection accuracy is equal to or higher than a second reference, and issue an alert in a case in which the detection accuracy is lower than the second reference.

In addition, in the information processing apparatus according to the present invention, the processor may derive a moving distance of the examination table based on the imaging range.

In addition, in the information processing apparatus according to the present invention, the processor may be configured to display a human body image imitating a human body on a display, and draw a start line of imaging and an end line of imaging based on the imaging range on the human body image.

In addition, in the information processing apparatus according to the present invention, the imaging range may be an imaging range in a case of capturing an image for positioning acquired before main imaging of the subject is performed.

In the information processing apparatus according to the present invention, the first detection model may have a smaller amount of calculation and a higher processing speed for detecting the feature points than the second detection model, and the second detection model may have a larger amount of calculation, a lower processing speed for detecting the feature points, and higher detection accuracy than the first detection model.

According to the present invention, there is provided an information processing method comprising: via a computer, acquiring a camera image generated by capturing a moving image of a subject on an examination table via a camera; selecting one detection model from among a plurality of detection models including a first detection model that places importance on a frame rate in a case of detecting the feature points and a second detection model that places importance on accuracy in a case of detecting the feature points, which are constructed so as to detect a plurality of feature points on the subject included in the camera image; detecting the plurality of feature points on the subject included in the camera image by using the selected detection model; and specifying an imaging range of the subject based on the plurality of feature points.

According to the present invention, there is provided an information processing program causing a computer to execute: a procedure of acquiring a camera image generated by capturing a moving image of a subject on an examination table via a camera; a procedure of selecting one detection model from among a plurality of detection models including a first detection model that places importance on a frame rate in a case of detecting the feature points and a second detection model that places importance on accuracy in a case of detecting the feature points, which are constructed so as to detect a plurality of feature points on the subject included in the camera image; a procedure of detecting the plurality of feature points on the subject included in the camera image by using the selected detection model; and a procedure of specifying an imaging range of the subject based on the plurality of feature points.

According to the present invention, in a case of setting the imaging range, the feature points can be appropriately detected according to the state of the subject.

### BRIEF DESCRIPTION OF THE DRAWINGS

Fig. 1 is a perspective view showing an outline of a CT apparatus to which an information processing apparatus according to a first embodiment of the present invention is applied.
Fig. 2 is a side view showing the CT apparatus to which the information processing apparatus according to the first embodiment is applied.
Fig. 3 is a view showing a schematic configuration of the information processing apparatus according to the first embodiment.
Fig. 4 is a view showing a functional configuration of an information processing apparatus according to the first embodiment.
Fig. 5 is a view showing feature points.
Fig. 6 is a view showing a schema in which a scan start line and a scan end line are displayed.
Fig. 7 is a flowchart showing processing performed in the first embodiment.
Fig. 8 is a view showing a functional configuration of an information processing apparatus according to a second embodiment.
Fig. 9 is a flowchart showing processing performed in the second embodiment.
Fig. 10 is a flowchart showing processing performed in a third embodiment.

### DETAILED DESCRIPTION

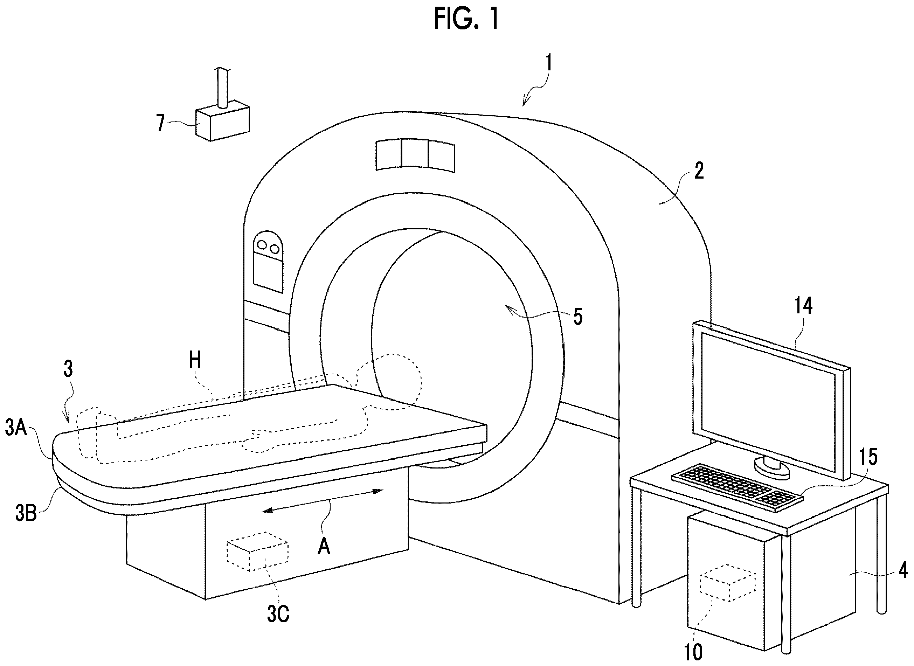

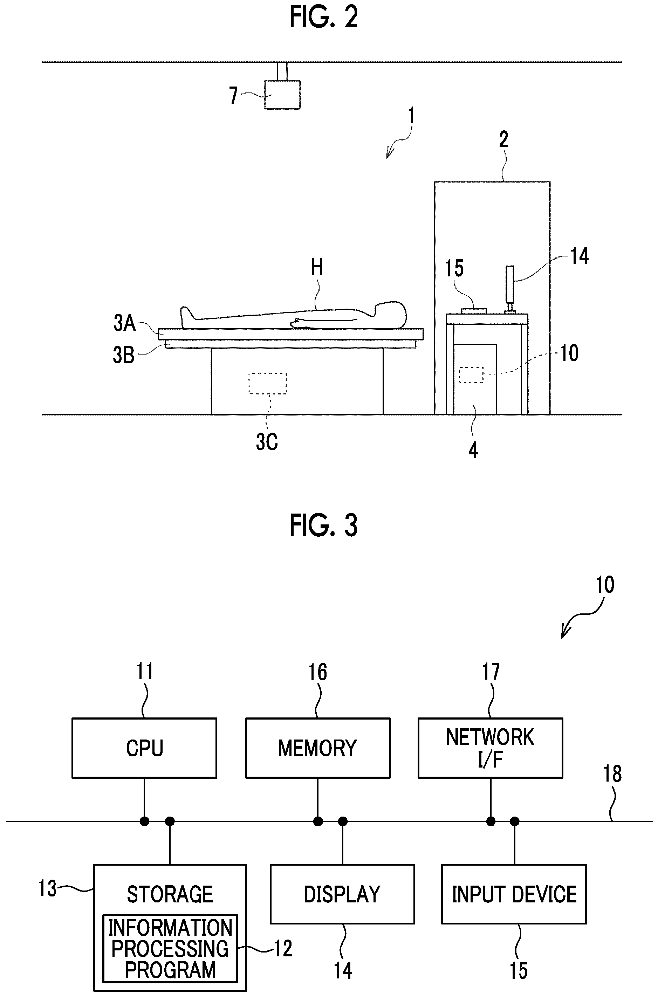

Hereinafter, description regarding embodiments of the present invention will be made with reference to the drawings. Fig. 1 is a perspective view showing an outline of a CT apparatus to which an information processing apparatus according to a first embodiment of the present invention is applied, and Fig. 2 is a side view showing the CT apparatus to which the information processing apparatus according to the first embodiment of the present invention is applied. As shown in Figs. 1 and 2, the CT apparatus 1 according to the present embodiment comprises a gantry 2, an examination table 3, and a console 4.

The gantry 2 has a tunnel-shaped structure with an opening portion 5 at the center thereof. Inside the gantry 2, a radiation source unit that emits X-rays and a detection unit that detects the X-rays to generate a radiation image are provided (neither of which is shown). The radiation source unit and the detection unit can rotate along an annular shape of the gantry 2 in a state in which a positional relationship in which the radiation source unit and the detection unit face each other is maintained. A controller that controls an operation of the CT apparatus 1 is provided inside the gantry 2.

The examination table 3 includes an examination table part 3A on which an subject lies down, a base part 3B that supports the examination table part 3A, and a driving part 3C that reciprocally moves the examination table part 3A in an arrow A direction. The examination table part 3A is slidable with respect to the base part 3B in the arrow A direction via the driving part 3C. In a case in which a CT image is captured, the examination table part 3A is slid, and a subject H lying down on the examination table part 3A is transported into the opening portion 5 of the gantry 2.

It should be noted that a camera 7 is installed above the examination table 3. The camera 7 is a camera that can capture a color image of RGB by detecting reflected light of the subject H. The camera 7 has an imaging element such as a lens and a charge coupled device (CCD), acquires a camera image, which is a moving image, by imaging the subject H on the examination table 3 at a predetermined frame rate, and outputs the camera image to the console 4. The camera 7 may be a camera in which an RGB camera and a near infrared (NIR) camera are integrated. In this case, the NIR camera is a stereo camera, and information in the depth direction of the subject H can be acquired by the parallax. By using the NIR camera of the camera 7 as the stereo camera, information in the depth direction of the subject H on the examination table 3 can be acquired. The NIR camera can perform imaging even in a case where the amount of light is insufficient. Therefore, in a case where the lightness of the examination room is insufficient for imaging of the RGB camera, the subject H may be imaged by the NIR camera.

The driving of the gantry 2, the driving of the examination table 3, and the imaging of the subject H via the camera 7 are performed in response to an input from the operator through the console 4. The console 4 includes the information processing apparatus according to the present embodiment.

Next, description regarding the information processing apparatus according to the first embodiment, which is included in the console 4 will be made. First, a hardware configuration of the information processing apparatus according to the first embodiment will be described with reference to Fig. 3. As shown in Fig. 3, an information processing apparatus 10 is a computer, such as a workstation, a server computer, and a personal computer, and comprises a central processing unit (CPU) 11, a non-volatile storage 13, and a memory 16 as a transitory storage area. The information processing apparatus 10 further comprises a display 14 such as a liquid crystal display, an input device 15 such as a keyboard and a mouse, and an interface such as a network interface (I/F) 17 connected to the CT apparatus 1. The CPU 11, the storage 13, the display 14, the input device 15, the memory 16, and the network I/F 17 are connected to a bus 18. It should be noted that the CPU 11 is an example of a processor of the present invention. The display 14 and the input device 15 are also shown in Figs. 1 and 2.

The storage 13 is realized by a hard disk drive (HDD), a solid state drive (SSD), a flash memory, and the like. The storage 13 as a storage medium stores an information processing program 12 installed in the information processing apparatus 10. The CPU 11 reads out the information processing program 12 from the storage 13, loads the readout information processing program 12 into the memory 16, and executes the loaded information processing program 12.

It should be noted that the information processing program 12 is stored in a storage device of a server computer connected to a network or in a network storage in a state of being accessible from the outside, and is downloaded and installed in the computer constituting the information processing apparatus 10 in response to a request. Alternatively, the information processing program 12 is distributed in a state of being recorded on a recording medium, such as a digital versatile disc (DVD) or a compact disc read only memory (CD-ROM), and is installed in the computer constituting the information processing apparatus 10 from the recording medium.

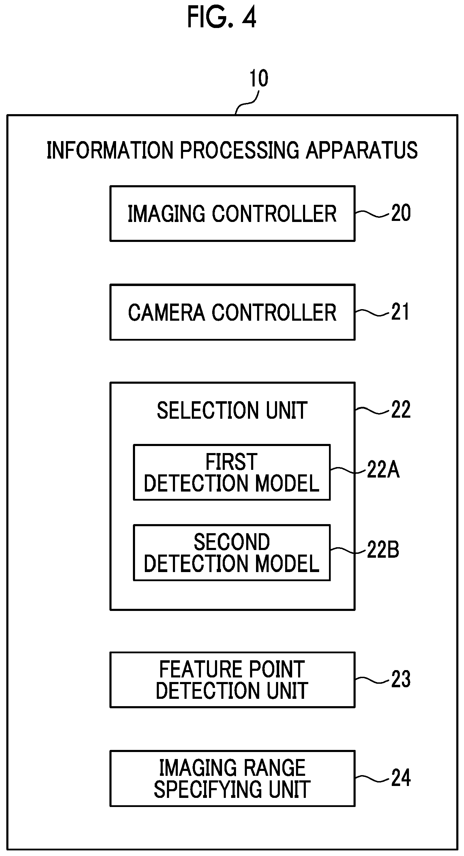

Next, description regarding a functional configuration of the information processing apparatus according to the first embodiment will be made. Fig. 4 is a view showing the functional configuration of the information processing apparatus according to the first embodiment. As shown in Fig. 4, the information processing apparatus 10 comprises an imaging controller 20, a camera controller 21, a selection unit 22, a feature point detection unit 23, and an imaging range specifying unit 24. The CPU 11 executes the information processing program 12, whereby the CPU 11 functions as the imaging controller 20, the camera controller 21, the selection unit 22, the feature point detection unit 23, and the imaging range specifying unit 24.

The imaging controller 20 controls an imaging unit, the detection unit, and the controller provided in the gantry 2 to perform the imaging of the subject H in response to an instruction from the input device 15. It should be noted that, in a case of CT imaging, scout imaging is performed before main imaging for acquiring a three-dimensional CT image in order to determine an imaging range. The scout imaging is performed by imaging the subject H, with the imaging unit and the detection unit being in a fixed state.

In a case of the scout imaging, as will be described below, the imaging range of the subject H is set, and the set imaging range is imaged by moving the examination table 3 to the opening portion 5 of the gantry 2 to perform the scout imaging. The scout image acquired by the scout imaging is a two-dimensional image including the imaging range set for the subject H. The display 14 displays the scout image. The operator views the scout image displayed on the display 14, thereby setting the imaging range in a case of performing the main imaging. After the imaging range is set, the operator inputs an instruction for the main imaging from the input device 15, so that the main imaging is performed and the three-dimensional CT image of the subject H is acquired. The storage 13 stores the acquired scout image and CT image.

The camera controller 21 controls the imaging of the subject H on the examination table 3 via the camera 7. The imaging of the subject H via the camera 7 is performed to set the imaging range in a case of performing the scout imaging. The imaging of the subject H via the camera 7 is performed from a preparation stage before the imaging. That is, the camera controller 21 starts the imaging via the camera 7 from a point in time before the subject H lies down on the examination table 3 in a supine position, and causes the camera 7 to acquire the camera image. Then, in a case in which the operator issues an instruction to start the scout imaging, the camera controller 21 stops the imaging via the camera 7. The acquired camera image is stored in the memory 16 in order to specify the imaging range described below.

The selection unit 22 selects one detection model from a first detection model 22A and a second detection model 22B, which are constructed to detect a plurality of feature points on the subject H included in the camera image acquired by the camera 7. The first detection model 22A is a detection model that places importance on the frame rate in a case where the feature points are detected. The second detection model 22B is a model that places importance on accuracy in detecting the feature points. The first detection model 22A and the second detection model 22B are constructed by training a neural network through machine learning.

The phrase "place importance on a frame rate" means that the processing speed for detecting the feature points is improved by, for example, reducing the amount of data to be processed, omitting the calculation, or the like. Since the first detection model 22A places importance on the frame rate, a model having a small amount of calculation and a high processing speed for detecting the feature points is used.

The phrase "place importance on accuracy" means that the accuracy in a case of detecting the feature points is improved without reducing the amount of data or omitting the calculation, although the calculation takes time. Since the importance is placed on the accuracy, the second detection model 22B has a large amount of calculation and has a lower processing speed than the first detection model 22A, but a model having higher accuracy of detecting the feature points than the first detection model 22A is used.

As the first detection model 22A, a neural network having a structure capable of performing processing of detecting the feature points at high speed with a small amount of calculation is used. The second detection model 22B uses a neural network having a structure having a larger amount of calculation but capable of detecting the feature points with higher accuracy than the first detection model 22A. For any neural network, a training image in which the entire human body, which is acquired by imaging the human body via the camera 7, is included and the 17 feature points are known is used for learning. It should be noted that the training image is acquired by, for example, imaging a person wearing an examination gown via the camera 7 in the same manner as in a case of actually performing an examination.

It should be noted that the neural network having the same structure may be used for the first detection model 22A and the second detection model 22B. In this case, the neural network is trained using different training images between the first detection model 22A and the second detection model 22B. For example, a first training image used for the training of the first detection model 22A has a relatively low resolution. On the other hand, as a second training image used for the training of the second detection model 22B, an image having a higher resolution than the first training image is used.

In the first embodiment, the selection unit 22 selects any one of the first detection model 22A or the second detection model 22B in accordance with the imaging part of the subject H. Here, the imaging parts of the subject H include a head, a chest, an abdomen, legs, and the entire body, but the head is likely to move during the imaging, and the chest or the abdomen is less likely to move during the imaging. Therefore, in a case in which the imaging part is the head or the entire body including the head, the selection unit 22 selects the first detection model 22A that places importance on the frame rate. On the other hand, in a case in which the imaging part is an abdomen or a leg, since imaging is often performed with a blanket covered thereon, the selection unit 22 selects the second detection model 22B that places importance on the accuracy and is more likely to detect the feature points.

In the present embodiment, a table in which the imaging part and the detection model to be selected are associated with each other is stored in the storage 13. The selection unit 22 selects a detection model according to the imaging part with reference to the table.

The imaging part of the subject H is included in the examination order provided by the doctor during the imaging. The operator refers to the examination order and sets the imaging part using the input device 15.

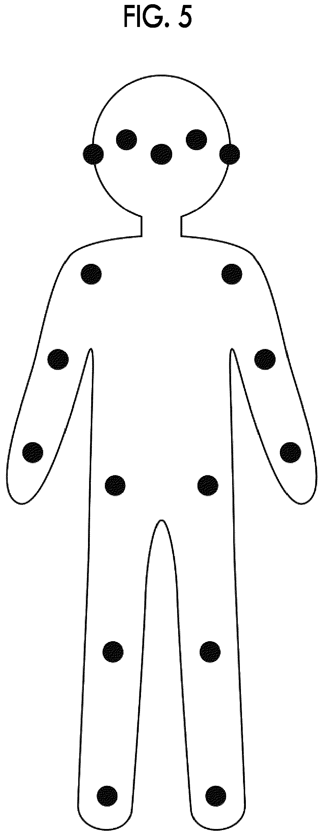

The feature point detection unit 23 detects the feature points from the camera image by using the detection model selected by the selection unit 22. Fig. 5 is a view showing the feature points. As shown in Fig. 5, in the present embodiment, the first and second detection models 22A and 22B are constructed to detect the 17 feature points on the subject H included in the camera image. The 17 feature points are both eyes, a nose, both ears, both shoulders, both elbows, both hands, both waists, both knees, and both feet. It should be noted that 18 feature points may be used by adding a point at the center of the clavicle to the 17 feature points.

The first detection model 22A and the second detection model 22B derive a probability representing a possibility of each of 17 feature points for each pixel of the camera image. The feature point detection unit 23 detects a pixel from which the highest probability is derived, as the feature point, for each of the 17 feature points. For example, in a case in which a right eye is detected as the feature point, the feature point detection unit 23 compares the probabilities of the right eye for all the pixels of the camera images derived by the first detection model 22A and the second detection model 22B, and detects the pixel having the highest probability as the feature point of the right eye.

The imaging range specifying unit 24 specifies the imaging range of the subject H in a case in which the scout imaging is performed based on the 17 feature points detected by using the feature point detection unit 23. Therefore, the imaging range specifying unit 24 first determines the detection accuracy of the feature points detected by using the feature point detection unit 23. As described above, the feature point detection unit 23 detects the pixel from which the highest probability is derived, as the feature point, for each of the 17 feature points. The detection accuracy is higher as the probability output by the detection model is higher for the detected feature point. Therefore, the imaging range specifying unit 24 compares a representative value of the probabilities derived by the detection model for the 17 feature points with a predetermined threshold value, determines that the detection accuracy is high in a case in which the representative value is equal to or higher than the threshold value, and determines that the detection accuracy is low in a case in which the representative value is lower than the threshold value.

As the representative value, an average value, a median value, a weighted average value corresponding to the imaging part, or the like can be used, but the present invention is not limited thereto. In addition, in the first detection model 22A that places importance on the frame rate and the second detection model 22B that places importance on the accuracy, even in a case where the same camera image is input, the probability derived for the feature point is smaller in the first detection model 22A. Therefore, in a case where the first detection model 22A is selected, a smaller threshold value than a threshold value used in a case where the second detection model 22B is selected may be used.

The imaging range specifying unit 24 performs processing of specifying the imaging range in a case in which it is determined that the detection accuracy is high. On the other hand, the imaging range specifying unit 24 performs alert display on the display 14 in a case in which it is determined that the detection accuracy is low. Here, in a case in which the subject H moves too much, a thick blanket is covered on the subject H, or the entire body of the subject H is not included in the imaging range of the camera 7, the feature points cannot be accurately detected regardless of which detection model is used, and the detection accuracy is deteriorated. In this case, the imaging range specifying unit 24 determines that the detection accuracy is low.

It should be noted that, in a case in which the alert display is performed, the operator manually sets the imaging range of the scout imaging. That is, the operator measures a distance from an initial position of the examination table 3 to a scan start line, further measures a distance between the scan start line and a scan end line, and inputs the measured distances from the input device 15. The movement of the examination table 3 during the scout imaging is controlled based on the input distances. The scan start line is an example of the start line of imaging, and the scan end line is an example of the end line of imaging.

Next, description regarding the processing of specifying the imaging range via the imaging range specifying unit 24 will be made. For example, in a case in which the imaging part is the head, the scout image is an image in which a range from the top of the head to the tip of the chin is the imaging range. Therefore, the imaging range specifying unit 24 sets a line connecting both eyes or both ears among the feature points detected by using the feature point detection unit 23, and further sets the scan start line at the top of the head and the scan end line between the chin and both shoulders. Then, a distance D1 between the scan start line and the scan end line is derived based on a relationship between the line connecting both the eyes or both the ears and the distance between the scan start line and the scan end line. A range of the distance D1 between the scan start line and the scan end line is the imaging range.

Here, before the scout imaging, the examination table 3 is at the initial position, and the subject H lies down on the examination table 3 in a supine position, so that a distance from an end part of the examination table 3 to the top of the head of the subject H can be known from the camera image. Therefore, the imaging range specifying unit 24 calculates a distance D2 from the end part of the examination table 3 to the top of the head of the subject H as a movement amount of the examination table 3 from the initial position to the scan start line, that is, a movement amount of the examination table 3 for the top of the head the subject H to reach the scan position in the CT apparatus 1.

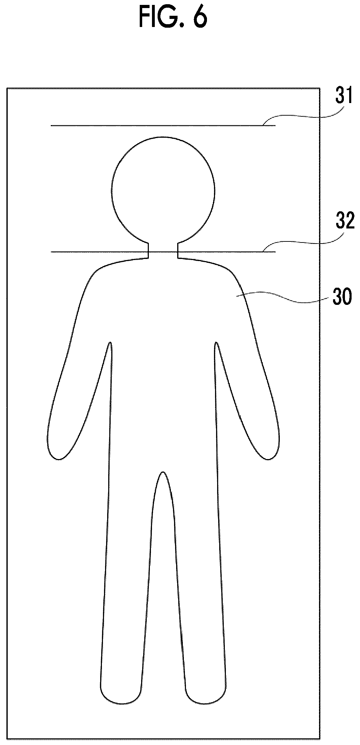

In a case in which the imaging range is specified, the imaging range specifying unit 24 displays the scan start line and the scan end line on a schema that is a human body diagram displayed on the display 14. Fig. 6 is a view showing a schema in which the start line and the end line are displayed. As shown in Fig. 6, a scan start line 31 and a scan end line 32 are shown in a schema 30. The operator checks the scan start line and the scan end line displayed on the display 14. After the check, in a case of OK, the operator issues the instruction to start the scout imaging by using the input device 15.

The information on the distances D1 and D2 is output to the CT apparatus 1 in response to the instruction to start the scout imaging. The imaging controller 20 controls the CT apparatus 1 such that the scan in the scout imaging is started after the driving part 3C moves the examination table 3 by the distance D2, and the scan in the scout imaging ends in a case in which the driving part 3C moves the examination table 3 by the distance D1.

The scout image acquired by the scout imaging is displayed on the display 14. The operator checks the scout image displayed on the display 14, and sets the imaging range of the main imaging on the scout image. Thereafter, the main imaging is performed by issuing an imaging instruction for the main imaging by using the input device 15, and the three-dimensional CT image of the imaging part of the subject H is acquired in the set imaging range of the main imaging.

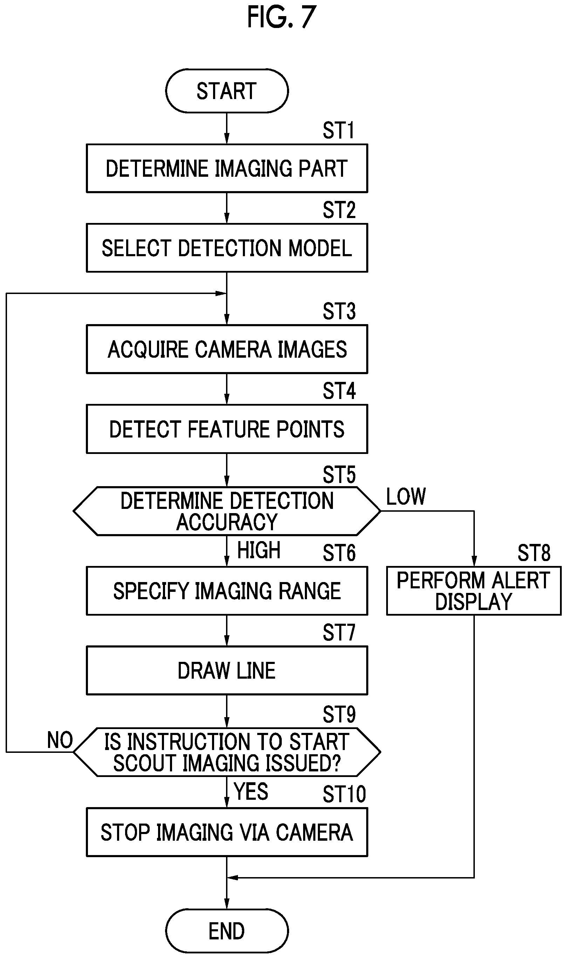

Next, description regarding processing performed in the first embodiment will be made. Fig. 7 is a flowchart showing the processing performed in the first embodiment. The processing is started in a case in which the imaging start instruction is issued from the input device 15, and the selection unit 22 determines the imaging part included in the examination order (step ST1). Then, the selection unit 22 selects any one of the first detection model 22A or the second detection model 22B in accordance with the determined imaging part (detection model selection; step ST2).

Then, the camera controller 21 starts the imaging via the camera 7 to acquire the camera image (step ST3), and the feature point detection unit 23 detects the feature points from the camera image by using the detection model selected by the selection unit 22 (step ST4). Then, the imaging range specifying unit 24 determines the detection accuracy of the feature points (step ST5). The imaging range specifying unit 24 specifies the imaging range during the scout imaging based on the feature points in a case in which it is determined that the detection accuracy is high (step ST6), and draws the scan start line and the scan end line during the scout imaging on the schema displayed on the display 14 based on the specified imaging range (line drawing; step ST7). On the other hand, in a case in which it is determined that the detection accuracy is low, the imaging range specifying unit 24 performs the alert display (step ST8). In a case in which the alert display is performed, the information processing apparatus 10 ends the imaging range specifying processing. In this case, the operator manually sets the imaging range of the scout imaging as described above.

Then, it is determined whether or not the instruction to start the scout imaging is issued by the operator (step ST9), the processing returns to step ST3 in a case in which a negative determination is made in step ST9, and the processing in and after step ST3 is repeated. In a case in which an affirmative determination is made in step ST9, the camera controller 21 stops the imaging via the camera 7 (step ST10), and the information processing apparatus 10 ends the imaging range specifying processing.

Thereafter, the scout image is acquired by performing the scout imaging via the imaging controller 20, and is displayed on the display 14. The operator checks the scout image, and then sets the imaging range of the main imaging. Then, the operator issues the instruction for the main imaging from the input device 15 to perform the main imaging, and the three-dimensional CT image of the subject H is acquired.

As described above, in the first embodiment, the detection model used for detecting the feature points is selected in accordance with the imaging part. Therefore, in a case where the imaging part is a head or the like which often moves during imaging, the first detection model 22A that places importance on the frame rate can be selected, and in a case where the imaging part is an abdomen or the like which does not often move during imaging but is often covered with a blanket or the like, the second detection model 22B that places importance on the accuracy can be selected. Therefore, in a case of setting the imaging range, the feature points can be appropriately detected in accordance with the imaging part, and as a result, the imaging range during the scout imaging can be appropriately specified by using the detected feature points.

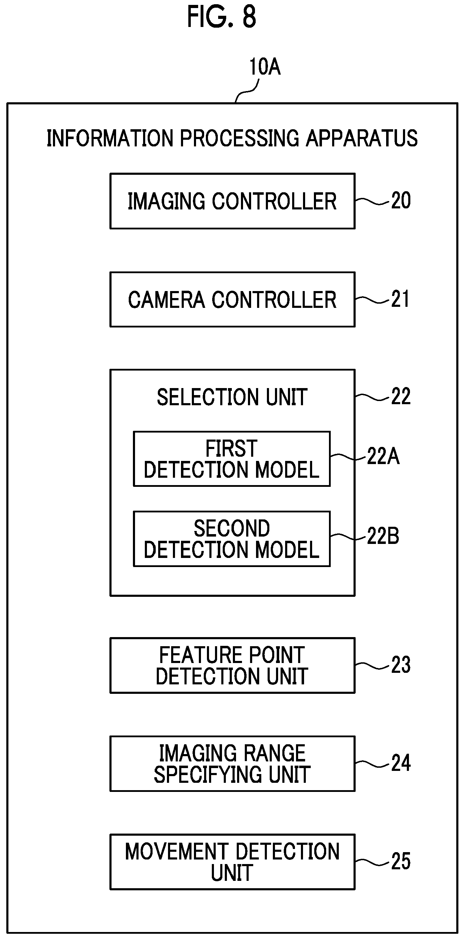

Next, description regarding a second embodiment of the present invention will be made. It should be noted that a hardware configuration of an information processing apparatus according to the second embodiment is the same as the hardware configuration of the information processing apparatus according to the first embodiment, and thus the detailed description thereof will be omitted here. Fig. 8 is a view showing a functional configuration of the information processing apparatus according to the second embodiment. In Fig. 8, the same reference numerals are applied to the same configurations as those in Fig. 4, and the detailed description thereof will be omitted. An information processing apparatus 10A according to the second embodiment is different from the information processing apparatus of the first embodiment in that the information processing apparatus 10A according to the second embodiment comprises a movement detection unit 25 that detects a movement of the subject H.

The movement detection unit 25 detects the movement of the subject H. Specifically, a two-dimensional movement of the feature points detected by using the feature point detection unit 23 is detected between frames that are adjacent in time to each other in the camera image. It should be noted that, in the second embodiment, the selection unit 22 first selects the first detection model 22A that places importance on the frame rate, and the feature point detection unit 23 detects the feature points by using the first detection model 22A. The feature points for detecting the movement of the subject H may be used in accordance with the imaging part. For example, in a case in which the imaging part is the head, the nose, both eyes, or both ears need only be used, and in a case in which the imaging part is the chest, both shoulders and left and right hip joints need only be used. It should be noted that a representative value of the movements of all the 17 feature points may be obtained as the movement.

The movement detection unit 25 determines that the movement is large in a case in which the detected movement is equal to or larger than a predetermined threshold value, and determines that the movement is small in a case in which the detected movement is smaller than the threshold value.

In the second embodiment, in a case where the movement detection unit 25 determines that the movement of the subject H is small, the selection unit 22 selects the second detection model 22B that places importance on the accuracy, instead of the first detection model 22A. The feature point detection unit 23 detects the feature points by using the second detection model 22B selected by the selection unit 22, and the imaging range specifying unit 24 specifies the imaging range by using the detected feature points. On the other hand, in a case in which the movement detection unit 25 determines that the movement of the subject H is large, the feature point detection unit 23 continues to use the first detection model 22A used to detect the feature points for detecting the movement to detect the feature points, and the imaging range specifying unit 24 specifies the imaging range by using the detected feature points.

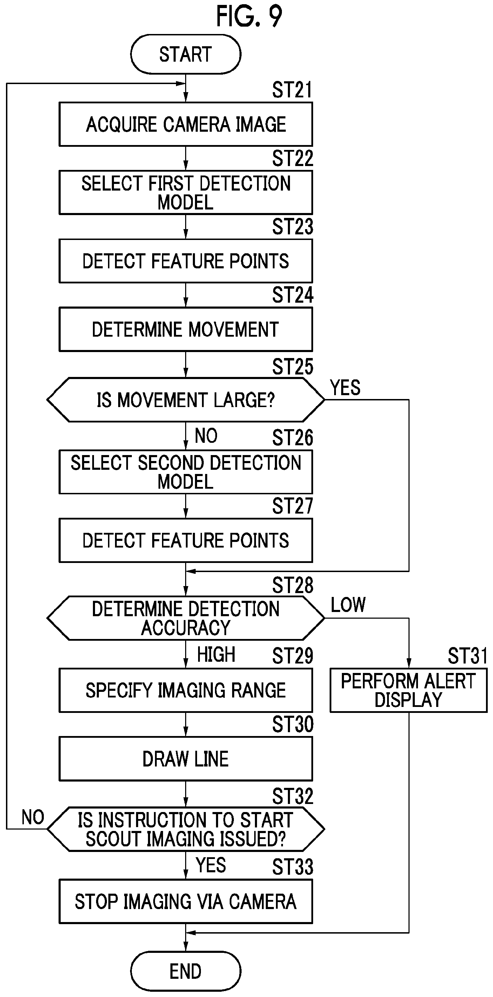

Next, description regarding processing performed in the second embodiment will be made. Fig. 9 is a flowchart showing the processing performed in the second embodiment. For example, the processing is started in response to the instruction to start the imaging from the input device 15, and the camera controller 21 starts the imaging via the camera 7 to acquire the camera image (step ST21). Next, the selection unit 22 selects the first detection model 22A that places importance on the frame rate (step ST22), and the feature point detection unit 23 detects the feature points from the camera image by using the detection model selected by the selection unit 22 (step ST23).

Then, the movement detection unit 25 detects the movement of the subject H by using the feature points detected by the feature point detection unit 23 (step ST24), and determines whether or not the movement is large (step ST25). In a case in which the movement of the subject H is small and a negative determination is made in step ST25, the selection unit 22 selects the second detection model 22B that places importance on the accuracy, instead of the first detection model 22A (step ST26). Then, the feature point detection unit 23 detects the feature points from the camera image (step ST27), and the imaging range specifying unit 24 determines the detection accuracy of the feature points (step ST28). In a case in which an affirmative determination is made in step ST25, the processing proceeds to step ST28, and the imaging range specifying unit 24 determines the detection accuracy of the feature points detected in step ST23.

The imaging range specifying unit 24 specifies the imaging range during the scout imaging based on the feature points in a case in which it is determined that the detection accuracy is high (step ST29), and draws the scan start line and the scan end line during the scout imaging on the schema displayed on the display 14 based on the specified imaging range (line drawing; step ST30). On the other hand, in a case in which it is determined that the detection accuracy is low, the imaging range specifying unit 24 performs the alert display (step ST31). In a case in which the alert display is performed, the information processing apparatus 10A ends the imaging range specifying processing. In this case, the operator manually sets the imaging range of the scout imaging as described above.

Then, it is determined whether or not the instruction to start the scout imaging is issued by the operator (step ST32), the processing returns to step ST21 in a case in which a negative determination is made in step ST32, and the processing in and after step ST21 is repeated. In a case in which an affirmative determination is made in step ST32, the camera controller 21 stops the imaging via the camera 7 (step ST33), and the information processing apparatus 10A ends the imaging range specifying processing.

As described above, in the second embodiment, the movement of the subject H is detected, the first detection model 22A that places importance on the frame rate is used in a case where the movement is large, and the second detection model 22B that places importance on the accuracy is used in a case where the movement is small, so that the feature points are detected. Therefore, in a case in which the imaging range is set, the feature point can be appropriately detected according to the movement of the subject, and as a result, the imaging range during the scout imaging can be appropriately specified by using the detected feature points.

Next, description regarding a third embodiment of the present invention will be made. It should be noted that a hardware configuration and a functional configuration of an information processing apparatus according to the third embodiment is the same as the hardware configuration and the functional configuration of the information processing apparatus according to the first embodiment, and thus the detailed description thereof will be omitted here. First, the third embodiment is different from the first embodiment in that the feature points are detected by the first detection model 22A that places importance on the frame rate, the detection accuracy of the feature points is determined, and the second detection model 22B that places importance on accuracy is used instead of the first detection model 22A in a case where the detection accuracy is low.

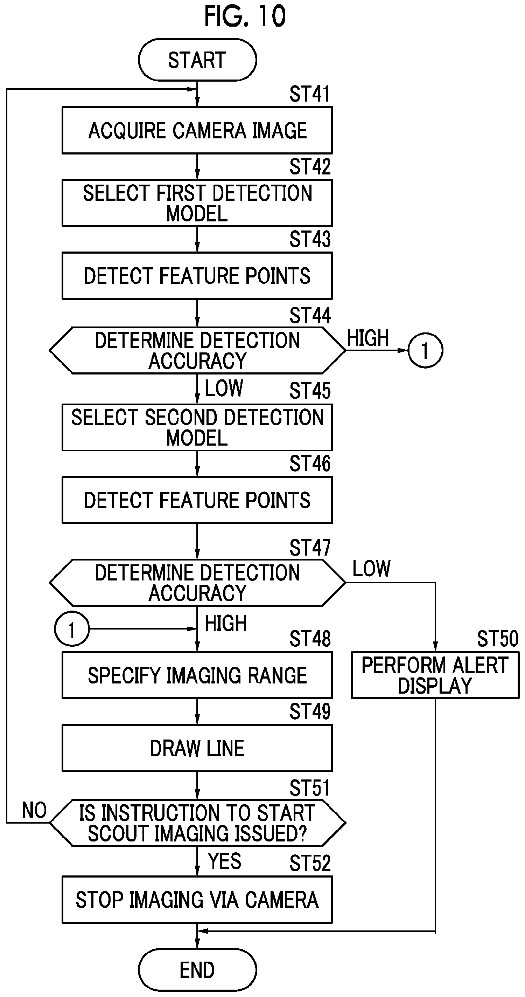

Next, description regarding processing performed in the third embodiment will be made. Fig. 10 is a flowchart showing the processing performed in the third embodiment. For example, the processing is started in response to the instruction to start the imaging from the input device 15, and the camera controller 21 starts the imaging via the camera 7 to acquire the camera image (step ST41). Next, the selection unit 22 selects the first detection model 22A that places importance on the frame rate (step ST42), and the feature point detection unit 23 detects the feature points from the camera image by using the detection model selected by the selection unit 22 (step ST43).

Next, the imaging range specifying unit 24 determines the detection accuracy of the feature points (step ST44). In a case in which the imaging range specifying unit 24 determines that the detection accuracy is low, the selection unit 22 selects the second detection model 22B that places importance on the accuracy, instead of the first detection model 22A (step ST45). Then, the feature point detection unit 23 detects the feature points from the camera image (step ST46), and the imaging range specifying unit 24 determines the detection accuracy of the feature points (step ST47).

The imaging range specifying unit 24 specifies the imaging range during the scout imaging based on the feature points in a case in which it is determined that the detection accuracy is high (step ST48), and draws the scan start line and the scan end line during the scout imaging on the schema displayed on the display 14 based on the specified imaging range (line drawing; step ST49). On the other hand, in a case in which it is determined that the detection accuracy is low, the imaging range specifying unit 24 performs the alert display (step ST50). In a case in which the alert display is performed, the information processing apparatus 10 ends the imaging range specifying processing. In this case, the operator manually sets the imaging range of the scout imaging as described above.

It should be noted that, in a case in which the imaging range specifying unit 24 determines that the detection accuracy is high in step ST44, the processing proceeds to the processing of step ST48, and the processing in and after step ST48 is performed.

Then, it is determined whether or not the instruction to start the scout imaging is issued by the operator (step ST51), the processing returns to step ST41 in a case in which a negative determination is made in step ST51, and the processing in and after step ST41 is repeated. In a case in which an affirmative determination is made in step ST51, the camera controller 21 stops the imaging via the camera 7 (step ST52), and the information processing apparatus 10 ends the imaging range specifying processing.

As described above, in the third embodiment, first, the feature points are detected by the first detection model 22A that places importance on the frame rate. Subsequently, in a case where the detection accuracy of the feature points is high, the first detection model 22A that places importance on the frame rate continues to be used to detect the feature points, and in a case where the detection accuracy of the feature points is low, the second detection model 22B that places importance on the accuracy is used to detect the feature points. Therefore, in a case of setting the imaging range, the feature points can be appropriately detected in accordance with the detection accuracy of the feature points, and as a result, the imaging range during the scout imaging can be appropriately specified by using the detected feature points.

In each of the above-described embodiments, the information processing apparatus according to the present invention is applied to the CT apparatus, but the present invention is not limited thereto. As long as an imaging apparatus acquires the scout image for setting the imaging range before the main imaging, the information processing apparatus according to the present invention may be applied to an MRI apparatus or the like.

In addition, in each of the above-described embodiments, the information processing apparatus comprises the imaging controller 20, but the present invention is not limited thereto. The imaging controller 20 may be provided separately from the information processing apparatus.

In addition, in each of the above-described embodiments, the detection model used for detecting the feature points is selected from the first detection model 22A and the second detection model 22B, but the present invention is not limited thereto. For example, a plurality of detection models having different processing speeds for detecting the feature points may be used for the detection models that place importance on the frame rate. In addition, a plurality of detection models having different accuracy of the feature point detection may be used for the detection models that place importance on the accuracy.

In addition, in the above-described embodiments, for example, as the hardware structure of the processing units that execute various types of processing, such as the imaging controller 20, the camera controller 21, the selection unit 22, the feature point detection unit 23, the imaging range specifying unit 24, and the movement detection unit 25, various processors described below can be used. As described above, the various processors include, in addition to the CPU that is a general-purpose processor which executes software (program) and functions as various processing units, a programmable logic device (PLD) that is a processor whose circuit configuration can be changed after manufacture, such as a field programmable gate array (FPGA), and a dedicated electrical circuit that is a processor having a circuit configuration which is designed for exclusive use in order to execute specific processing, such as an application specific integrated circuit (ASIC).

One processing unit may be configured by one of these various processors, or may be configured by combining two or more processors of the same type or different types (for example, by combining a plurality of FPGAs or combining the CPU and the FPGA). A plurality of processing units may be configured by one processor.

As an example of configuring the plurality of processing units by one processor, first, as represented by a computer of a client, a server, and the like there is a form in which one processor is configured by combining one or more CPUs and software and this processor functions as the plurality of processing units. Second, as represented by a system on a chip (SoC) or the like, there is a form of using a processor that realizes the function of the entire system including the plurality of processing units by one integrated circuit (IC) chip. In this way, as the hardware structure, the various processing units are configured by using one or more of the various processors described above.

Further, as the hardware structures of these various processors, more specifically, an electrical circuit (circuitry) in which circuit elements such as semiconductor elements are combined can be used.

### Explanation of References

1: CT apparatus
2: gantry
3: examination table
3A: examination table part
3B: base part
3C: driving part
4: console
5: opening portion
7: camera
10, 10A: information processing apparatus
11: CPU
12: information processing program
13: storage
14: display
15: input device
16: memory
17: network I/F
18: bus
20: imaging controller
21: camera controller
22: selection unit
23: feature point detection unit
24: imaging range specifying unit
25: movement detection unit
H: subject

## Claims

1. An information processing apparatus comprising:
at least one processor (11),
wherein the processor
acquires a camera image generated by capturing a moving image of a subject on an examination table via a camera (7),
selects one detection model from among a plurality of detection models including a first detection model that places importance on a frame rate in a case of detecting the feature points and a second detection model that places importance on accuracy in a case of detecting the feature points, which are constructed so as to detect a plurality of feature points on the subject included in the camera image,
detects the plurality of feature points on the subject included in the camera image by using the selected detection model, and
specifies an imaging range of the subject based on the plurality of feature points.

2. The information processing apparatus according to claim 1,
wherein the processor selects the detection model in accordance with an imaging part of the subject.

3. The information processing apparatus according to claim 1 or 2,
wherein the processor determines detection accuracy of the feature points, specifies the imaging range based on the feature points in a case in which the detection accuracy is equal to or higher than a reference, and issues an alert in a case in which the detection accuracy is lower than the reference.

4. The information processing apparatus according to claim 1,
wherein the processor detects a movement of the subject based on the camera image, specifies the imaging range based on the feature points detected by using the first detection model in a case in which the movement of the subject is equal to or larger than a reference, and specifies the imaging range based on the feature points detected by using the second detection model in a case in which the movement of the subject is smaller than the reference.

5. The information processing apparatus according to claim 3,
wherein the processor detects the feature points from the camera image by selecting the first detection model, and detects a movement of the subject based on the feature points.

6. The information processing apparatus according to claim 4 or 5,
wherein the processor determines detection accuracy of the feature points, specifies the imaging range based on the feature points in a case in which the detection accuracy is equal to or higher than a reference, and issues an alert in a case in which the detection accuracy is lower than the reference.

7. The information processing apparatus according to claim 1,
wherein the processor detects the feature points from the camera image by selecting the first detection model, determines detection accuracy of the feature points, specifies the imaging range based on the feature points detected by using the first detection model in a case in which the detection accuracy is equal to or higher than a first reference, selects the second detection model in a case in which the detection accuracy is lower than the first reference, and specifies the imaging range based on the feature points detected by using the second detection model.

8. The information processing apparatus according to claim 7,
wherein the processor determines detection accuracy of the feature points detected by using the second detection model, specifies the imaging range based on the feature points in a case in which the detection accuracy is equal to or higher than a second reference, and issues an alert in a case in which the detection accuracy is lower than the second reference.

9. The information processing apparatus according to any one of claims 1 to 8,
wherein the processor derives a moving distance of the examination table based on the imaging range.

10. The information processing apparatus according to any one of claims 1 to 9,
wherein the processor displays, on a display (14), a human body image imitating a human body and draws, on the human body image, a start line of imaging and an end line of imaging based on the imaging range.

11. The information processing apparatus according to any one of claims 1 to 10,
wherein the imaging range is an imaging range in a case of capturing an image for positioning acquired before main imaging of the subject is performed.

12. The information processing apparatus according to any one of claims 1 to 11,
wherein the first detection model has a smaller amount of calculation and a higher processing speed for detecting the feature points than the second detection model, and the second detection model has a larger amount of calculation, a lower processing speed for detecting the feature points, and higher detection accuracy than the first detection model.

13. An information processing method comprising:
via a computer,
acquiring a camera image generated by capturing a moving image of a subject on an examination table via a camera (7);
selecting one detection model from among a plurality of detection models including a first detection model that places importance on a frame rate in a case of detecting the feature points and a second detection model that places importance on accuracy in a case of detecting the feature points, which are constructed so as to detect a plurality of feature points on the subject included in the camera image;
detecting the plurality of feature points on the subject included in the camera image by using the selected detection model; and
specifying an imaging range of the subject based on the plurality of feature points.

14. A computer-readable storage medium that stores an information processing program causing a computer to execute:
a procedure of acquiring a camera image generated by capturing a moving image of a subject on an examination table via a camera (7);
a procedure of selecting one detection model from among a plurality of detection models including a first detection model that places importance on a frame rate in a case of detecting the feature points and a second detection model that places importance on accuracy in a case of detecting the feature points, which are constructed so as to detect a plurality of feature points on the subject included in the camera image;
a procedure of detecting the plurality of feature points on the subject included in the camera image by using the selected detection model; and
a procedure of specifying an imaging range of the subject based on the plurality of feature points.

## Patentansprüche

1. Informationsverarbeitungsvorrichtung, umfassend:
mindestens einen Prozessor (11),
wobei der Prozessor
ein Kamerabild, das durch Aufnehmen eines Bewegtbildes einer Untersuchungsperson auf einem Untersuchungstisch via eine Kamera (7) erzeugt wird, erfasst,
ein Detektionsmodell aus mehreren Detektionsmodellen auswählt, die ein erstes Detektionsmodell, das in einem Fall des Detektierens der Merkmalspunkte eine Bildrate priorisiert, und ein zweites Detektionsmodell, das in einem Fall des Detektierens der Merkmalspunkte Genauigkeit priorisiert, enthalten, die so konstruiert sind, dass sie mehrere Merkmalspunkte auf der Untersuchungsperson, die in dem Kamerabild enthalten sind, detektieren,
die mehreren Merkmalspunkte auf der Untersuchungsperson, die in dem Kamerabild enthalten sind, durch Verwenden des ausgewählten Detektionsmodells detektiert, und
einen Bildgebungsbereich der Untersuchungsperson auf der Grundlage der mehreren Merkmalspunkte spezifiziert.

2. Informationsverarbeitungsvorrichtung nach Anspruch 1,
wobei der Prozessor das Detektionsmodell in Übereinstimmung mit einem Bildgebungsteil der Untersuchungsperson auswählt.

3. Informationsverarbeitungsvorrichtung nach Anspruch 1 oder 2,
wobei der Prozessor Detektionsgenauigkeit der Merkmalspunkte bestimmt, den Bildgebungsbereich auf der Grundlage der Merkmalspunkte in einem Fall, in dem die Detektionsgenauigkeit gleich oder höher als eine Referenz ist, spezifiziert und in einem Fall, in dem die Detektionsgenauigkeit niedriger als die Referenz ist, eine Warnung ausgibt.

4. Informationsverarbeitungsvorrichtung nach Anspruch 1,
wobei der Prozessor eine Bewegung der Untersuchungsperson auf der Grundlage des Kamerabildes detektiert, den Bildgebungsbereich auf der Grundlage der Merkmalspunkte, die durch Verwenden des ersten Detektionsmodells detektiert worden sind, in einem Fall, in dem die Bewegung der Untersuchungsperson gleich oder größer als eine Referenz ist, spezifiziert und den Bildgebungsbereich auf der Grundlage der Merkmalspunkte, die durch Verwenden des zweiten Detektionsmodells detektiert worden sind, in einem Fall, in dem die Bewegung der Untersuchungsperson kleiner als die Referenz ist, spezifiziert.

5. Informationsverarbeitungsvorrichtung nach Anspruch 3,
wobei der Prozessor die Merkmalspunkte aus dem Kamerabild durch Auswählen des ersten Detektionsmodells detektiert und eine Bewegung der Untersuchungsperson auf der Grundlage der Merkmalspunkte detektiert.

6. Informationsverarbeitungsvorrichtung nach Anspruch 4 oder 5,
wobei der Prozessor Detektionsgenauigkeit der Merkmalspunkte bestimmt, den Bildgebungsbereich auf der Grundlage der Merkmalspunkte in einem Fall, in dem die Detektionsgenauigkeit gleich oder höher als eine Referenz ist, spezifiziert und in einem Fall, in dem die Detektionsgenauigkeit niedriger als die Referenz ist, eine Warnung ausgibt.

7. Informationsverarbeitungsvorrichtung nach Anspruch 1,
wobei der Prozessor die Merkmalspunkte aus dem Kamerabild durch Auswählen des ersten Detektionsmodells detektiert, Detektionsgenauigkeit der Merkmalspunkte bestimmt, den Bildgebungsbereich auf der Grundlage der Merkmalspunkte, die durch Verwenden des ersten Detektionsmodells detektiert worden sind, in einem Fall, in dem die Detektionsgenauigkeit gleich oder höher als eine erste Referenz ist, spezifiziert, das zweite Detektionsmodell in einem Fall, in dem die Detektionsgenauigkeit niedriger als die erste Referenz ist, auswählt und den Bildgebungsbereich auf der Grundlage der Merkmalspunkte, die durch Verwenden des zweiten Detektionsmodells detektiert worden sind, spezifiziert.

8. Informationsverarbeitungsvorrichtung nach Anspruch 7,
wobei der Prozessor Detektionsgenauigkeit der Merkmalspunkte, die durch Verwenden des zweiten Detektionsmodells detektiert worden sind, bestimmt, den Bildgebungsbereich auf der Grundlage der Merkmalspunkte in einem Fall, in dem die Detektionsgenauigkeit gleich oder höher als eine zweite Referenz ist, spezifiziert und eine Warnung in einem Fall, in dem die Detektionsgenauigkeit niedriger als die zweite Referenz ist, ausgibt.

9. Informationsverarbeitungsvorrichtung nach einem der Ansprüche 1 bis 8,
wobei der Prozessor eine Bewegungsentfernung des Untersuchungstisches auf der Grundlage des Bildgebungsbereichs ableitet.

10. Informationsverarbeitungsvorrichtung nach einem der Ansprüche 1 bis 9,
wobei der Prozessor ein menschliches Körperbild, das einen menschlichen Körper imitiert, auf einer Anzeige (14) anzeigt und eine Bildgebungsstartlinie und eine Bildgebungsendlinie auf der Grundlage des Bildgebungsbereichs auf dem menschlichen Körperbild zeichnet.

11. Informationsverarbeitungsvorrichtung nach einem der Ansprüche 1 bis 10,
wobei der Bildgebungsbereich ein Bildgebungsbereich in einem Fall des Aufnehmens eines Bildes zum Positionieren ist, das erfasst wird, bevor Hauptbildgebung der Untersuchungsperson durchgeführt wird.

12. Informationsverarbeitungsvorrichtung nach einem der Ansprüche 1 bis 11,
wobei das erste Detektionsmodell einen kleineren Rechenaufwand und eine höhere Verarbeitungsgeschwindigkeit zum Detektieren der Merkmalspunkte als das zweite Detektionsmodell aufweist und das zweite Detektionsmodell einen größeren Rechenaufwand, eine niedrigere Verarbeitungsgeschwindigkeit zum Detektieren der Merkmalspunkte und eine höhere Detektionsgenauigkeit als das erste Detektionsmodell aufweist.

13. Informationsverarbeitungsverfahren, umfassend:
via einen Computer,
Erfassen eines Kamerabildes, das durch Aufnehmen eines Bewegtbildes einer Untersuchungsperson auf einem Untersuchungstisch via eine Kamera (7) erzeugt wird;
Auswählen eines Detektionsmodells aus mehreren Detektionsmodellen, die ein erstes Detektionsmodell, das in einem Fall des Detektierens der Merkmalspunkte eine Bildrate priorisiert, und ein zweites Detektionsmodell, das in einem Fall des Detektierens der Merkmalspunkte Genauigkeit priorisiert, enthalten, die so konstruiert sind, dass sie mehrere Merkmalspunkte auf der Untersuchungsperson, die in dem Kamerabild enthalten sind, detektieren;
Detektieren der mehreren Merkmalspunkte auf der Untersuchungsperson, die in dem Kamerabild enthalten sind, durch Verwenden des ausgewählten Detektionsmodells; und
Spezifizieren eines Bildgebungsbereichs der Untersuchungsperson auf der Grundlage der mehreren Merkmalspunkte.

14. Computerlesbares Speichermedium, das ein Informationsverarbeitungsprogramm speichert, das einen Computer veranlasst, auszuführen:
ein Verfahren des Erfassens eines Kamerabildes, das durch Aufnehmen eines Bewegtbildes einer Untersuchungsperson auf einem Untersuchungstisch via eine Kamera (7) erzeugt wird;
ein Verfahren des Auswählens eines Detektionsmodells aus mehreren Detektionsmodellen, die ein erstes Detektionsmodell, das in einem Fall des Detektierens der Merkmalspunkte eine Bildrate priorisiert, und ein zweites Detektionsmodell, das in einem Fall des Detektierens der Merkmalspunkte Genauigkeit priorisiert, enthalten, die so konstruiert sind, dass sie mehrere Merkmalspunkte auf der Untersuchungsperson, die in dem Kamerabild enthalten sind, detektieren;
ein Verfahren des Detektierens der mehreren Merkmalspunkte auf der Untersuchungsperson, die in dem Kamerabild enthalten sind, durch Verwenden des ausgewählten Detektionsmodells; und
ein Verfahren des Spezifizierens eines Bildgebungsbereichs der Untersuchungsperson auf der Grundlage der mehreren Merkmalspunkte.

## Revendications

1. Appareil de traitement d'informations comprenant :
au moins un processeur (11),
dans lequel le processeur
acquiert une image de caméra générée en capturant une image en mouvement d'un sujet sur une table d'examen via une caméra (7),
sélectionne un modèle de détection parmi une pluralité de modèles de détection incluant un premier modèle de détection qui accorde de l'importance à une fréquence d'images dans un cas de détection des points caractéristiques et un deuxième modèle de détection qui accorde de l'importance à la précision dans un cas de détection des points caractéristiques, qui sont construits de manière à détecter une pluralité de points caractéristiques sur le sujet inclus dans l'image de caméra,
détecte la pluralité de points caractéristiques sur le sujet inclus dans l'image de caméra en utilisant le modèle de détection sélectionné, et
spécifie une plage d'imagerie du sujet sur la base de la pluralité de points caractéristiques.

2. Appareil de traitement d'informations selon la revendication 1,
dans lequel le processeur sélectionne le modèle de détection en fonction d'une partie d'imagerie du sujet.

3. Appareil de traitement d'informations selon la revendication 1 ou la revendication 2, dans lequel le processeur détermine une précision de détection des points caractéristiques, spécifie la plage d'imagerie sur la base des points caractéristiques dans un cas où la précision de détection est égale ou supérieure à une référence, et émet une alerte dans un cas où la précision de détection est inférieure à la référence.

4. Appareil de traitement d'informations selon la revendication 1,
dans lequel le processeur détecte un mouvement du sujet sur la base de l'image de caméra, spécifie la plage d'imagerie sur la base des points caractéristiques détectés en utilisant le premier modèle de détection dans un cas où le mouvement du sujet est égal ou supérieur à une référence, et spécifie la plage d'imagerie sur la base des points caractéristiques détectés en utilisant le deuxième modèle de détection dans un cas où le mouvement du sujet est inférieur à la référence.

5. Appareil de traitement d'informations selon la revendication 3,
dans lequel le processeur détecte les points caractéristiques à partir de l'image de caméra en sélectionnant le premier modèle de détection, et détecte un mouvement du sujet sur la base des points caractéristiques.

6. Appareil de traitement d'informations selon la revendication 4 ou la revendication 5, dans lequel le processeur détermine une précision de détection des points caractéristiques, spécifie la plage d'imagerie sur la base des points caractéristiques dans un cas où la précision de détection est égale ou supérieure à une référence, et émet une alerte dans un cas où la précision de détection est inférieure à la référence.

7. Appareil de traitement d'informations selon la revendication 1,
dans lequel le processeur détecte les points caractéristiques à partir de l'image de caméra en sélectionnant le premier modèle de détection, détermine la précision de détection des points caractéristiques, spécifie la plage d'imagerie sur la base des points caractéristiques détectés en utilisant le premier modèle de détection dans un cas où la précision de détection est égale ou supérieure à une première référence, sélectionne le deuxième modèle de détection dans un cas où la précision de détection est inférieure à la première référence, et spécifie la plage d'imagerie sur la base des points caractéristiques détectés en utilisant le deuxième modèle de détection.

8. Appareil de traitement d'informations selon la revendication 7,
dans lequel le processeur détermine la précision de détection des points caractéristiques détectés en utilisant le deuxième modèle de détection, spécifie la plage d'imagerie sur la base des points caractéristiques dans un cas où la précision de détection est égale ou supérieure à une deuxième référence, et émet une alerte dans un cas où la précision de détection est inférieure à la deuxième référence.

9. Appareil de traitement d'informations selon l'une quelconque des revendications 1 à 8, dans lequel le processeur dérive une distance de déplacement de la table d'examen sur la base de la plage d'imagerie.

10. Appareil de traitement d'informations selon l'une quelconque des revendications 1 à 9, dans lequel le processeur affiche, sur un affichage (14), une image de corps humain imitant un corps humain et trace, sur l'image de corps humain, une ligne de début d'imagerie et une ligne de fin d'imagerie sur la base de la plage d'imagerie.

11. Appareil de traitement d'informations selon l'une quelconque des revendications 1 à 10,
dans lequel la plage d'imagerie est une plage d'imagerie dans un cas de capture d'une image de positionnement acquise avant que l'imagerie principale du sujet ne soit effectuée.

12. Appareil de traitement d'informations selon l'une quelconque des revendications 1 à 11,
dans lequel le premier modèle de détection présente une quantité de calcul plus faible et une vitesse de traitement plus élevée pour détecter les points caractéristiques que le deuxième modèle de détection, et le deuxième modèle de détection présente une quantité de calcul plus élevée, une vitesse de traitement plus faible pour détecter les points caractéristiques et une précision de détection plus élevée que le premier modèle de détection.

13. Procédé de traitement d'informations comprenant :
via un ordinateur,
acquérir une image de caméra générée en capturant une image en mouvement d'un sujet sur une table d'examen via une caméra (7) ;
sélectionner un modèle de détection parmi une pluralité de modèles de détection incluant un premier modèle de détection qui accorde de l'importance à une fréquence d'images dans un cas de détection des points caractéristiques et un deuxième modèle de détection qui accorde de l'importance à la précision dans un cas de détection des points caractéristiques, qui sont construits de manière à détecter une pluralité de points caractéristiques sur le sujet inclus dans l'image de caméra ;
détecter la pluralité de points caractéristiques sur le sujet inclus dans l'image de caméra en utilisant le modèle de détection sélectionné ; et
spécifier une plage d'imagerie du sujet sur la base de la pluralité de points caractéristiques.

14. Support de stockage lisible par ordinateur qui stocke un programme de traitement d'informations amenant un ordinateur à exécuter :
une procédure consistant à acquérir une image de caméra générée en capturant une image en mouvement d'un sujet sur une table d'examen via une caméra (7) ;
une procédure consistant à sélectionner un modèle de détection parmi une pluralité de modèles de détection incluant un premier modèle de détection qui accorde de l'importance à une fréquence d'images dans un cas de détection des points caractéristiques et un deuxième modèle de détection qui accorde de l'importance à la précision dans un cas de détection des points caractéristiques, qui sont construits de manière à détecter une pluralité de points caractéristiques sur le sujet inclus dans l'image de caméra ;
une procédure consistant à détecter la pluralité de points caractéristiques sur le sujet inclus dans l'image de caméra en utilisant le modèle de détection sélectionné ; et
une procédure consistant à spécifier une plage d'imagerie du sujet sur la base de la pluralité de points caractéristiques.
